# EUROPÄISCHE PATENTANMELDUNG

(11) **EP 3 825 699 A1**
(43) Veröffentlichungstag der Anmeldung: **26.05.2021**
(21) Anmeldenummer: 20212375.8
(22) Anmeldetag: 24.01.2012
(51) Int. Cl.: G01N 35/02, G01N 35/00, C12M 1/36, C12M 1/22, C12M 1/00, C12M 3/00, G01N 35/04

(54) **VORRICHTUNG ZUR LAGERUNG UND HANDHABUNG VON PETRISCHALEN, LAGERVORRICHTUNG UND LAGERSCHACHT FÜR LABOROBJEKTE**

(30) Priorität: 28.01.2011 CH 1512011; 06.05.2011 CH 7832011
(62) Teilanmeldung aus: 12000411.4
(71) Anmelder: LICONIC AG, 9493 Mauren (LI)
(72) Erfinder: Malin, Cosmas, 9493 Mauren (LI)
(74) Vertreter: E. Blum & Co. AG

(57) **Zusammenfassung**

Die Vorrichtung umfasst eine Lagervorrichtung (1), eine Inspektionsvorrichtung (2), eine Transfervorrichtung (3), eine Übergabestation (4) sowie eine Umschlagvorrichtung (5). Mit der Umschlagvorrichtung (5) werden Petrischalen (15) zwischen der Lagervorrichtung (1) und der Übergabestation (4) transferiert, mit der Transfervorrichtung (3) zwischen der Übergabestation (4) und der Inspektionsvorrichtung (2). Die Transfervorrichtung (3) hebt den in der Übergabestation (4) oben liegenden Boden (48) der Petrischalen an und wendet diesen über dem Deckel (49), so dass allfällige Tropfen vom Boden (48) in den Deckel (49) fallen. Dann fährt die Transfervorrichtung (3) den Boden (48) zur Inspektionsvorrichtung, wo er inspiziert wird. In der Lagervorrichtung (1) werden die Petrischalen in Lagerschächten (14) übereinander gelagert. Die Lagerschächte (14) sind in Kreisen angeordnet und besitzen abgeschrägte hintere Eckbereiche, so dass eine kompakte Lagerung möglich ist. Die Lagerschächte (14) sind im Bodenbereich mit Stiften (71) positioniert und werden von oben mit Zungen (73) niedergehalten.

## Beschreibung

### Gebiet der Erfindung

Die Erfindung betrifft eine Vorrichtung zur Lagerung und Handhabung von Laborobjekten, insbesondere Petrischalen. Weiter betrifft die Erfindung eine Lagervorrichtung und einen Lagerschacht sowie eine Laborvorrichtung.

### Hintergrund

Petrischalen sind flache, runde, durchsichtige Schalen mit übergreifendem Deckel, die in der Biologie, Medizin oder Chemie verbreitet zum Einsatz kommen. Dabei werden Petrischalen zur Kultivierung von Mikroorganismen und Zellkulturen genutzt. Eine flache Schicht aus einem gelförmigen Nährmedium ist in die Petrischale gefüllt und versorgt die wachsenden Mikroorganismen mit Wasser und den notwendigen Nährstoffen.

Anwendungsspezifische Mikroorganismen werden lokal in das Nährmedium eingebracht. Anschliessend werden die Petrischalen meist mit dem Deckel nach unten und dem Nährboden nach oben inkubiert. Bei dieser Lagerung lastet das Gewischt der Platte auf dem Deckel, wodurch der Verschluss zwischen Deckel und Schale verbessert wird. Überschüssiges Wasser bildet sich nicht auf dem Nährboden, sondern sammelt bevorzugt sich auf dem Deckel.

Während der Inkubationszeit wird das Wachstum der Kulturen mehrmals visuell inspiziert. In Anwendungen in denen mit grossen Zahlen von Platten gearbeitet wird, besteht Bedarf für eine Automatisierung des Vorgangs. Dabei wird nach bestimmten zeitlichen Vorgaben jeweils eine Petrischale dem Inkubator bzw. der Lagervorrichtung entnommen und einer Inspektionsvorrichtung zugeführt. Für die Inspektion sollen der Inspektionsvorrichtung die Platten mit dem Nährboden nach unten zugeführt werden.

Zum Transferieren der Petrischalen zwischen Lagervorrichtung und Inspektionsvorrichtung wird eine Transfervorrichtung benötigt. Üblicherweise besitzt diese einen Schwenkarm, der an einer Wandung der Inkubationskammer bzw. Lagervorrichtung angeordnet ist. Eine Petrischale wird von einem Vakuumsauger, der am äusseren Ende des Schwenkarms angeordnet ist, erfasst und mittels einer Drehbewegung von 180 Grad aus dem Inkubator entnommen und gleichzeitig gedreht. Bei der Inspektionsvorrichtung muss der Vakuumsauger für die Inspektion entfernt werden.

Weiter sind zur Lagerung von Laborobjekten Lagerschächte bekannt, welche jeweils Platz für mehrere Laborobjekte übereinander bieten.

US 2006/0289371 beschreibt eine Lagervorrichtung gemäss Oberbegriff der unabhängigen Ansprüche.

### Darstellung der Erfindung

In einem ersten Aspekt stellt sich die Aufgabe, eine Lagervorrichtung bereitzustellen, deren Lagerschächte einfach ausgetauscht werden können.

Gemäss diesem Aspekt betrifft die Erfindung eine Lagervorrichtung zur Aufnahme von Laborobjekten, insbesondere von Petrischalen oder Mikrotiterplatten. Die Lagervorrichtung soll eine einfache Montage und Demontage der Lagerschächte erlauben. Gemäss Anspruch besitzt die Lagervorrichtung mindestens einen Lagerschacht, wobei jeder Lagerschacht mehrere Lagerplätze zur Aufnahme jeweils eines Laborobjekts übereinander aufweist. Am Boden jedes Lagerschachts ist mindestens eine Öffnung angeordnet, in welche mindestens ein Stift einer Unterlage des Lagerschachts eingreift. Von oben sind die Lagerschächte mittels einer federnden Zunge gehalten. Durch Deformation der Zunge kann ein Lagerschacht angehoben, von den Stiften gelöst und der Lagervorrichtung entnommen werden.

In einem zweiten Aspekt betrifft die Erfindung einen Lagerschacht zur Aufnahme von Petrischalen, der insbesondere (aber nicht nur) zum Einsatz in der oben beschriebenen Vorrichtung geeignet ist und eine kompakte Lagerung erlauben soll. Der Lagerschacht besitzt eine Rückwand und zwei Seitenwände. Zumindest an den Seitenwänden, optional auch an der Rückwand, sind Auflagen für die periphere Aufnahme der Petrischalen vorgesehen. Die Rückwand geht über zwei Übergangsbereiche in die beiden Seitenwände über. Jeder Übergangsbereich ist gerundet, oder er verläuft schräg zur Rückwand und zur jeweiligen angrenzenden Seitenwand. Diese Ausgestaltung, welche die runde Bauform von Petrischalen berücksichtigt, hat den Vorteil, dass die Lagerschächte eng in einem Kreis, beispielsweise auf einem Karussell angeordnet werden können, so dass eine Lagerung mit hoher räumlicher Kapazität möglich ist.

Weiter betrifft die Erfindung eine Vorrichtung zur Lagerung und Handhabung von Petrischalen, wobei die Petrischalen einen Boden und einen Deckel aufweisen, wobei an einem Umfang des Bodens eine Bodenwand angeordnet ist. Die Vorrichtung umfasst:
- Eine Lagervorrichtung zum Lagern der Petrischalen mit dem Deckel nach unten;
- Eine Inspektionsvorrichtung zum automatisierten Inspizieren von Petrischalen ohne Deckel und
- Eine Transfervorrichtung zum Transferieren der Petrischalen zwischen der Lagervorrichtung und der Inspektionsvorrichtung.

Die Transfervorrichtung weist einen Greifer auf, mit welchem jeweils eine Petrischale ohne den Deckel lateral an der Bodenwand ergreifbar sind, und
- wobei die Lagervorrichtung gemäss dem ersten Aspekt der Erfindung ausgeführt sind und/oder
- wobei sie einen Lagerschacht gemäss dem zweiten Aspekt der Erfindung aufweist.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine Ansicht einer Anlage von oben,
Fig. 2 eine Ansicht der Transfervorrichtung und der angrenzenden Komponenten,
Fig. 3 einen Teil der Transfervorrichtung,
Fig. 4 eine Ausführung eines Greifers,
Fig. 5 eine von mehreren Auflagen der Übergabestation,
Fig. 6 einen Lagerschacht für Petrischalen,
Fig. 7 ein Detail (Kreis A) des Lagerschachts von Fig. 6,
Fig. 8 eine Befestigungsvariante für die Lagerschächte,
Fig. 9 eine Transfervorrichtung mit zwei Übergabestationen übereinander;
Fig. 10 eine alternative Ausführung zum Greifer nach Fig. 4;
Figur 11 eine Darstellung einer weiteren Transfervorrichtung mit zwei Übergabestationen zur Erläuterung weiterer bevorzugter Ausführungen;
Figur 12 eine weitere Ausführung eines Greifers in schaubildlicher Ansicht;
Figur 13 den Greifer von Figur 12 in Draufsicht von oben;
Figur 14 eine Ansicht einer bevorzugten Variante der Vorrichtung und eines Teils einer damit zusammenwirkenden Laborvorrichtung;
Figur 15 eine schaubildliche Ansicht einer Hebeeinrichtung; und
Figur 16 eine weitere Ausführung der Lagervorrichtung.

### Wege zur Ausführung der Erfindung

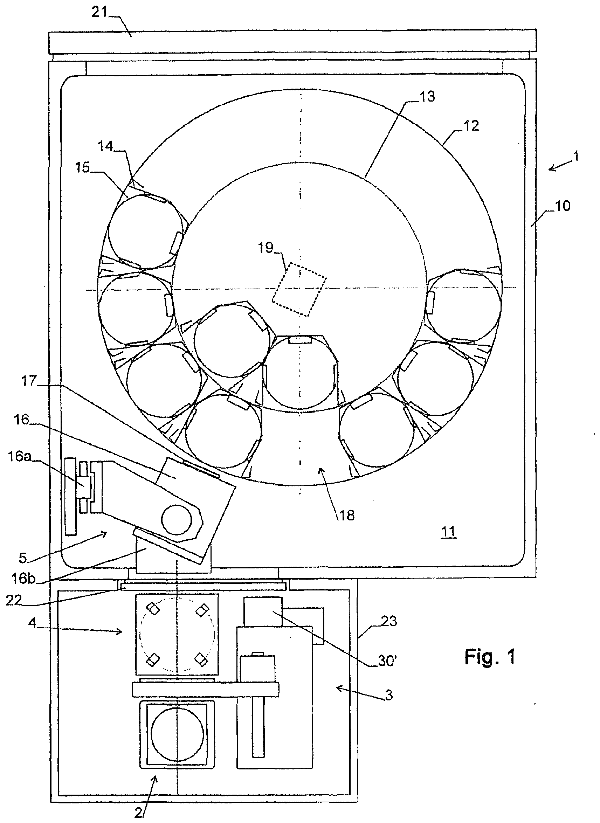

Fig. 1 zeigt eine Vorrichtung zur Lagerung und Handhabung von Petrischalen von oben. Sie umfasst eine Lagervorrichtung 1, eine Inspektionsvorrichtung 2, eine Transfervorrichtung 3, eine Übergabestation 4 sowie eine Umschlagvorrichtung 5. Der Aufbau und die Funktion dieser Komponenten werden im Folgenden im Detail beschrieben.

Die Lagervorrichtung 1 ist vorzugsweise als Inkubator, d.h. als Klimaschrank, ausgestaltet, in dessen Inneren mittels einer geeigneten Klimatisierung eine gewünschte Temperatur und Atmosphäre aufrecht erhalten werden kann. Sie besitzt eine Wand 10, welche einen Innenraum 11 umschliesst. Im Innenraum 11 sind konzentrisch zwei unabhängig voneinander drehbare Karusselle 12 und 13 angeordnet, welche jeweils einen Kreis von Lagerschächten 14 tragen, von denen in Fig. 1 nur ein Teil dargestellt ist. Jeder Lagerschacht 14 bildet eine Vielzahl von Lagerplätzen für Petrischalen 15 übereinander.

In einer Ecke des Innenraums 11 ist weiter die Umschlagvorrichtung 5 angeordnet, die dazu dient, die Petrischalen 15 zwischen den Lagerplätzen und der Transfervorrichtung 3 hin- und her zu transportieren. Hierzu besitzt die Umschlagvorrichtung mindestens einen an einer Liftsäule 16a vertikal verfahrbaren Wagen 16, an welchem eine horizontal ausfahrbare und um eine vertikale Achse schwenkbare Schaufel 17 angeordnet ist. Diese Schaufel kann in der in Fig. 1 gezeigten Schwenkstellung in die Lagerschächte 14 eingefahren werden, um jeweils eine Petrischale 15 aufzunehmen oder abzulegen. Durch eine Lücke 18 im äusseren Kreis der Lagerschächte 14 kann die Schaufel auch auf die Lagerplätze der Lagerschächte des inneren Kreises zugreifen. Hierzu wird Karussell 12 mit dem äusseren Kreis der Lagerschächte 14 so gedreht, dass die Lücke 18 im Bereich der Schaufel 17 zu liegen kommt. Optional kann in der Mitte des inneren Karussells 13 noch eine Hilfseinrichtung 19 vorgesehen sein, welche die Arbeit der Schaufel 17 unterstützt und ihr hilft, die Lagerplätze des inneren Kreises zu beschicken.

Wie in Fig. 1 weiter angedeutet, sind an der Liftsäule 16a in der gezeigten Ausführung mindestens zwei Wagen 16, 16b übereinander angeordnet. In Fig. 1 ist dabei der untere Wagen 16b momentan zur Übergabestation 4 hin gedreht. Durch Verwendung mehrer Wagen 16, 16b, die unabhängig voneinander vertikal verfahrbar sind, kann die Umschlagkapazität erhöht werden. Der untere Wagen 16b bedient die untere Hälfte der Lagervorrichtung 1, und der obere Wagen 16 die obere Hälfte.

Wie weiter ersichtlich, besitzt die Lagervorrichtung 1 in der dargstellten Ausführung eine Benutzertüre 21 sowie eine automatisch öffen- und schliessbare Hilfstüre 22. Um mit der Umschlagvorrichtung 5 eine Petrischale vom Innenraum 11 nach aussen oder in umgekehrter Richtung zu transportieren, wird die Hilfstüre 22 geöffnet.

Ausserhalb der Lagervorrichtung 1 kann z.B. ein Tisch 23 angeordnet sein, der die im Folgenden beschriebenen Komponenten trägt. Der Tisch 23 kann an der Aussenseite der Lagervorrichtung 1 befestigt sein.

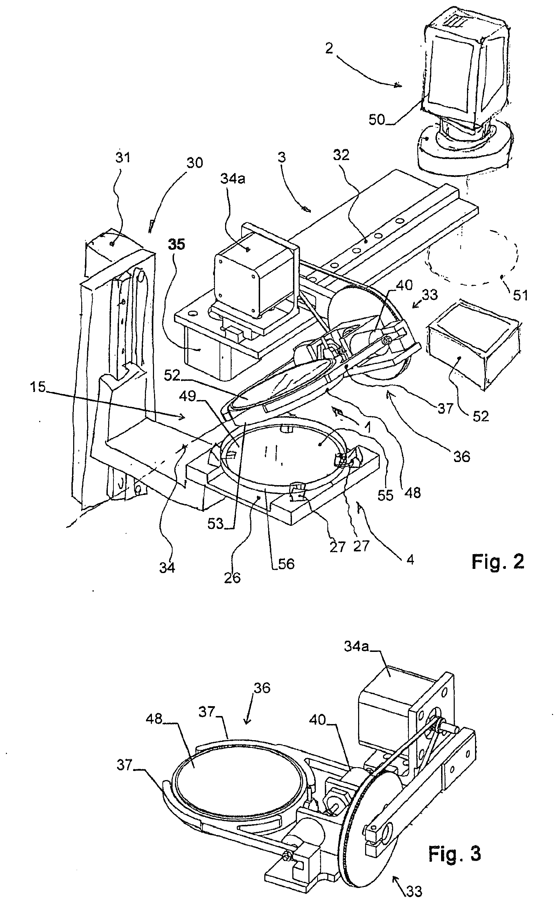

Die Übergabestation 4, welche auch in Fig. 2 dargestellt ist, dient dazu, mindestens eine von der Umschlagvorrichtung 5 ausgetragene Petrischale zu übernehmen, oder temporär mindestens eine Petrischale aufzunehmen, welche sodann von der Umschlagvorrichtung 5 angehoben und in einen der Lagerplätze der Lagervorrichtung 1 abgelegt wird.

Wie in Fig. 2 dargestellt, umfasst die Übergabestation 4 einen Transfertisch 26, auf welchem vier gegenüber dem Transfertisch 26 erhöhte Auflagen 27 angeordnet sind, auf denen eine in der Übergabestation 4 abgelegte Petrischale ruht.

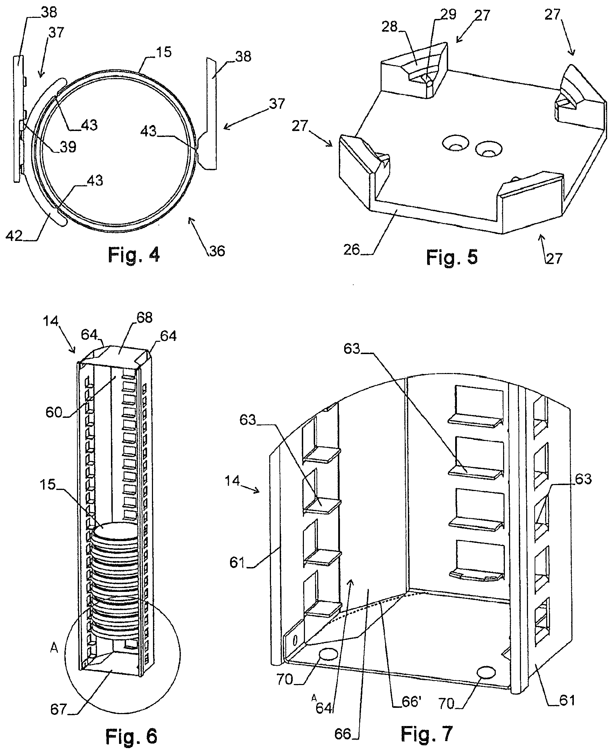

Fig. 5 zeigt den Transfertisch 26 mit vier Auflagen 27 im Detail. Wie ersichtlich, bildet jede Auflage 27 eine seitliche, schräg verlaufende Auflauffläche 28, an welcher die Petrischale zentriert wird. An die Auflauffläche 28 schliesst ein Auflagepolster 29 an, auf dem die Petrischale ruht. Das Auflagepolster 29 ist vorzugsweise aus einem Material mit hoher Haftreibung (d.h. mit höherer Haftreibung als das restliche Material der Auflage 27), so z.B. aus Gummi, so dass die darauf abgelegte Petrischale (bzw. deren Deckel) bei den unten beschriebenen Manipulationen sich möglichst nicht verschiebt.

In der Ausführung nach Fig. 2 ist die Übergabestation 4 vertikal verfahrbar an einem Lift 30 mit Antrieb 31 angeordnet. Demgegenüber ist die Übergabestation 4 in der Ausführung nach Fig. 1 nicht höhenverstellbar - vielmehr ist die Transfervorrichtung 3 an einem Lift 30' höhenverstellbar angeordnet. Beide Varianten sind möglich. Wichtig ist, dass die Transfervorrichtung 3 (bzw. deren im Folgenden beschriebene Greifer) vertikal gegenüber der Übergabestation 4 bewegt werden kann.

Die Transfervorrichtung 3 besitzt eine Schiene 32, entlang der ein Kopf 33 entlang einer horizontalen Achse 34 verfahren werden kann. Hierzu ist ein Horizontalantrieb 35 vorgesehen.

Der Kopf 33, dessen Aufbau aus Fig. 2 und 3 ersichtlich ist, ist um eine Achse parallel zur Achse 34 schwenkbar, wozu ein Wendeantrieb 34a vorgesehen ist, und trägt einen Greifer 36 mit zwei Fingern 37. Der Greifer 36 ist somit um eine horizontale Achse schwenkbar und vertikal gegenüber der Übergabestation 4 beweglich.

Der Greifer 36 besitzt einen Fingerantrieb 40, mit welchem die beiden Finger 37 gegeneinander bewegt werden können, z.B. in einer linearen Bewegung.

Fig. 4 zeigt einen möglichen Aufbau eines Greifers 36 im Detail. Jeder Finger 37 besitzt einen Schaft 38, wobei die beiden Schafte 38 gegeneinander bewegt werden können. An den Fingern 37 sind mindestens drei gegeneinander gerichtete Erhöhungen 43 vorgesehen, um die Seitenwände der Petrischale 15 zu erfassen. Diese dienen dazu, die Wand der Petrischale 15 an definierten Punkten (bzw. entlang definierten Mantellinien) zu beaufschlagen, so dass die Petrischale 15 sicher und ohne Spiel aufgenommen werden kann.

Vorzugsweise ist mindestens eine der Erhöhungen 43 federnd am jeweiligen Finger 37 befestigt. In der Ausführung nach Fig. 4 ist hierzu an einem der Finger 37 eine als Blattfeder ausgestaltete Federung 39 angeordnet, welche ihrerseits ein gebogenes Basisteil 42 hält. Am Basisteil 42 ist mindestens eine der Erhöhungen 43 angeordnet. Im gezeigten Beispiel sind zwei Erhöhungen 43 am Basisteil 42 angeordnet.

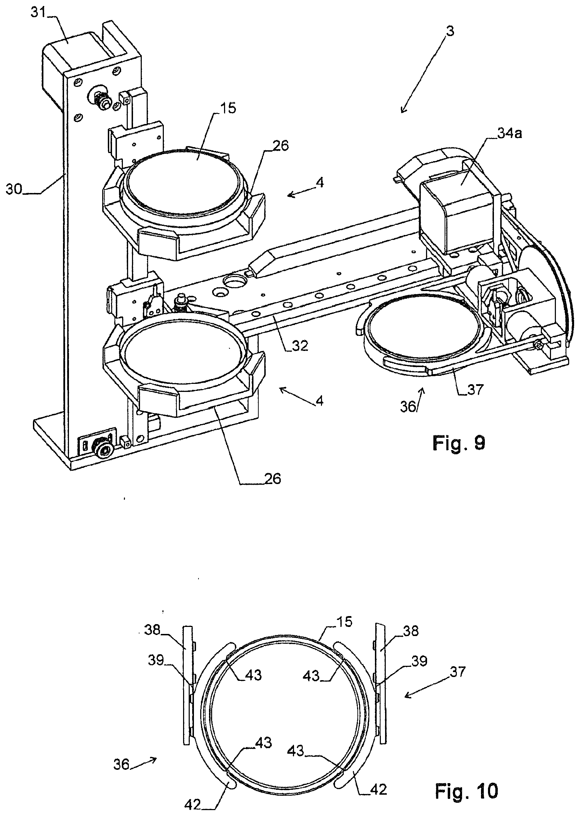

Fig. 10 zeigt eine alternative Ausführung des Greifers 36, bei welcher beide Finger 37 je eine Federung 39 aufweisen, die jeweils einen Basisteil 42 trägt. In diesem Falle ist jeder Basisteil 42 mit jeweils zwei Erhöhungen 43 ausgestattet, da die Federungen 39 ein gewisses Verkippen der Basisteile 42 zulassen und somit auch vier Erhöhungen 43 spielfrei gegen eine nicht ganz runde Petrischale 15 gedrückt werden können.

Um die Funktion der vorliegenden Vorrichtung zu verstehen, ist zunächst der Aufbau der Petrischalen 15 zu erläutern. Eine solche Petrischale ist in Fig. 2 sichtbar. Sie besitzt einen Boden 48 und einen Deckel 49. Wie eingangs erwähnt, befindet sich die zu untersuchende Probe am Boden 48, z.B. in einem am Boden 48 klebenden Nährsubstrat, aber die Petrischale wird in der Lagervorrichtung so gelagert, dass der Boden 48 oberhalb des Deckels 49 ist, d.h. auf dem Deckel 49 aufliegt. Zur Analyse der Petrischale in der im Folgenden noch genauer beschriebenen Inspektionsvorrichtung muss der Boden 48 vom Deckel 49 getrennt und um 180° um eine horizontale Achse geschwenkt werden.

Der Boden 48 besitzt eine runde Bodenfläche 52, an deren Umfang eine periphere, ungefähr zylindrische Bodenwand 53 vorgesehen ist. Der Deckel 49 besitzt seinerseits ebenfalls eine runde Deckelfläche 55, an deren Umfang eine zylindrische Deckelwand 56 vorgesehen ist. Der Innendurchmesser der Deckelwand 56 ist etwas grösser als der Aussendurchmesser der Bodenwand 53. Sind Boden 48 und Deckel 49 zusammengefügt, so greift die Bodenwand 53 in die Deckelwand 56 ein und ruht gegen die Deckelfläche 55.

Wie erwähnt, sind die Petrischalen 15 in den Lagerplätzen der Lagervorrichtung 1 mit dem Deckel 49 nach unten gelagert. Die Umschlagvorrichtung transferiert die Petrischalen 15 ohne sie zu wenden zwischen den Lagerplätzen und der Übergabestation 4. Somit kommen die Petrischalen mit dem Deckel 49 nach unten in der Übergabestation 4 zu liegen. Nun wird der Greifer 36 von oben über den Boden 48 der Petrischale 15 gelegt. Die Finger 37 werden aufeinander zugefahren, so dass sie den Boden 48 der Petrischale 15 an der Bodenwand 53 ergreifen. Jetzt kann der Greifer 36 relativ zur Übergabestation 4 angehoben werden, so dass er den Boden 48 aus dem Deckel 49 zieht. Falls sich zu diesem Zeitpunkt ein Tropfen vom Boden 48 löst, so fällt er in den Deckel.

Nun wird der Greifer 36 mit der aufgenommenen Petrischale gewendet. Dies geschieht vertikal oberhalb des in der Übergabestation 4 liegenden Deckels 49, so dass auch in dieser Phase allfällige sich vom Boden 48 lösende Tropfen in den Deckel 49 fallen. Vorzugsweise wird der Boden 48 nicht von oberhalb des Deckels 49 weg bewegt, bis er um mehr als 90° gewendet wurde.

Nun kann der Greifer 36 mit dem aufgenommenen und gewendeten Deckel 48 mit Hilfe des Horizontalantriebs 35 zur Inspektionsvorrichtung 2 verfahren werden. In der vorliegenden Ausführung handelt es sich um eine optische Inspektionsvorrichtung mit einer Kamera 50, welche ein Bild einer in einer Messposition 51 angeordneten Petrischale zu liefern vermag. Die Messposition 51 befindet sich zwischen der Kamera 50 und einer Lichtquelle 52, um Aufnahmen im Durchlicht zu erzeugen.

Mit dem Greifer 36 kann der aufgenommene und gewendete Deckel 48 zur Messposition 51 verfahren werden. Hierzu wird der Horizontalantrieb 35 verwendet, mit welchem der Kopf 33 entlang der Schiene 32 bewegt werden kann. Dabei ist die Vorrichtung so ausgestaltet, dass der Deckel 48 der Petrischale während der Inspektion durch die Inspektionsvorrichtung mit dem Greifer 36 gehalten wird. Es ist also nicht notwendig, dass der Deckel 48 in der Inspektionsvorrichtung 2 abgelegt wird. Dadurch wird der Vorgang beschleunigt. Da der Greifer den Deckel 48 nur an der Peripherie angreift, stört er den Inspektionsprozess nicht.

Wie bereits erwähnt, sind die Petrischalen 15 in der Lagervorrichtung 1 in Lagerschächten 14 gelagert. Ein vorteilhafter Lagerschacht ist in Fig. 6 und 7 dargestellt.

Der gezeigte Lagerschacht ist im Wesentlichen aus einem einzigen Blech geformt, welches in geeigneter Weise zugeschnitten und gebogen wurde. Insbesondere bildet das Blech eine vertikale Rückwand 60 und zwei vertikale Seitenwände 61. Zumindest an den Seitenwänden 61, vorzugsweise auch an der Rückwand 60, sind seitliche Ablagen 63 zur Aufnahme der Petrischalen angeordnet. Diese werden von abgebogenen Zungen des Blechs der Rück- und Seitenwände gebildet.

Die Rückwand 60 geht jeweils über einen Übergangsbereich 64 in jede Seitenwand 61 über. Dieser Übergangsbereich verläuft in der Ausführung nach Fig. 6 und 7 schräg zur Seiten, so dass Volumen, welches im rückseitigen Bereich der Lagerschächte 14 von den runden Petrischalen 15 nicht benötigt wird, freigegeben werden kann. Dies erlaubt eine dichtere Packung der Lagerschächte 14 in einem Kreis, wie in Fig. 1 illustriert, indem die Lagerschächte im Kreis näher zusammenrücken können.

Da Petrischalen einen typischen Durchmesser in der Grössenordnung von 9 cm besitzen, kann der Übergangsbereich 64 einen ebenen, vertikal verlaufenden Abschnitt 66 besitzen, dessen Ausdehnung senkrecht zur Längsachse (Vertikalachse) des Lagerschachts 14 mindestens 2 cm beträgt.

Da Petrischalen rund sind, kann der Übergangsbereich 64 grundsätzlich auch gerundet verlaufen, wie dies in Fig. 7 unter Bezugsziffer 66' gestrichelt dargestellt ist. In diesem Falle ist, wenn der Krümmungsradius des Übergangsbereichs 64 ungefähr jenem der Petrischalen entspricht, eine noch dichtere Packung der Lagerschächte 14 möglich, allerdings bei etwas höherem Herstellungsaufwand. In diesem Falle kann der Krümmungsradius des Übergangsbereichs im Mittel mindestens 2 cm betragen, vorzugsweise mindestens 5 cm.

Wie aus Fig. 6 und 7 weiter ersichtlich, gehen auch der Boden 67 und die Decke 68 jedes Lagerschachts 14 einstückig in die Rückwand 60 über und sind durch Biegen aus dem gleichen Blech wie die Rückwand 60 und die Seitenwände 61 gefertigt.

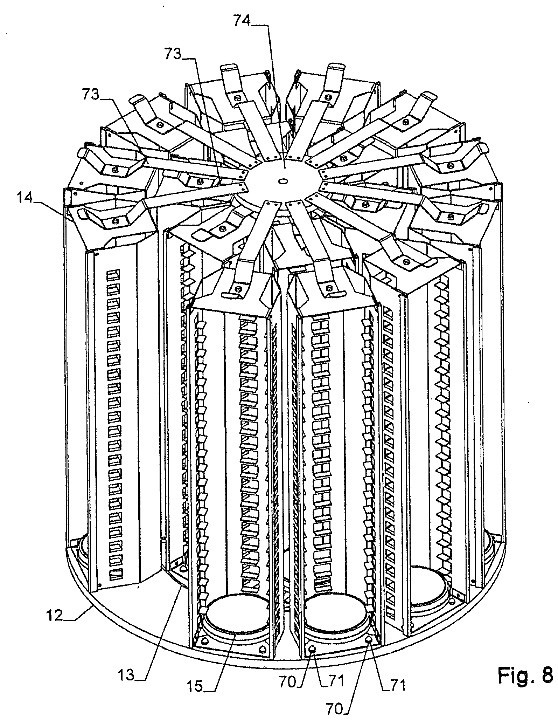

Die Lagerschächte 14 können in der Lagervorrichtung 1 grundsätzlich in jeder geeigneten Art befestigt werden. Eine bevorzugte Befestigungsvariante ist in Fig. 8 dargestellt. Hier sind im Boden 67 eine order mehrere Öffnungen 70 vorgesehen (vgl. Fig. 7), in welche Stifte 71 eingreifen. Die Stifte 71 sind mit der Unterlage verbunden, auf welcher der jeweilige Lagerschacht 14 steht. In der Ausführung nach Fig. 8 wird diese Unterlage von einem der Karussells 12 oder 13 gebildet. Die Stifte 71 sind nach oben konisch zulaufend.

Von der Oberseite werden die Lagerschächte von federnden, radial verlaufenden Zungen 73 gehalten, die an einem Mittelbereich 74 der Lagervorrichtung 1 befestigt sind. Die Zungen 73 sind vorteilhaft federnd vorgespannt und drücken von oben auf die Lagerschächte 14. Die Zungen 73 können mit den Lagerschächten 14 verschraubt sein.

Fig. 9 zeigt eine zweite Ausführung der Transfervorrichtung 3. Sie unterscheidet sich von jener gemäss Fig. 1 dadurch, dass zwei Übergabestationen 4 mit jeweils einem eigenen Transfertisch 26 übereinander vorgesehen sind. Diese sind am Lift 30 vertikal verfahrbar, vorzugsweise mit einem gemeinsamen Liftantrieb 31. Jede der Übergabestationen 4 kann auf die Höhe gefahren werden, in welcher sie mit dem Greifer 36 und der Umschlagvorrichtung 5 zusammenwirken kann. Somit kann z.B. eine erste Petrischale analysiert werden, während ihr Deckel in der einen Übergabestation 4 liegt, während die Umschlagvorrichtung 5 der anderen Übergabestation 4 einen Petrischale entnimmt oder ihr eine Petrischale zuführt.

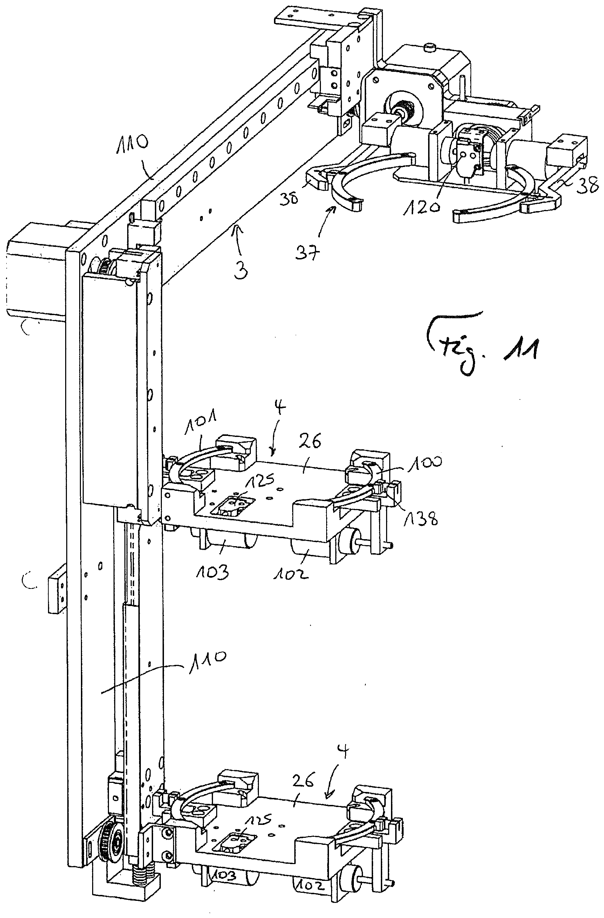

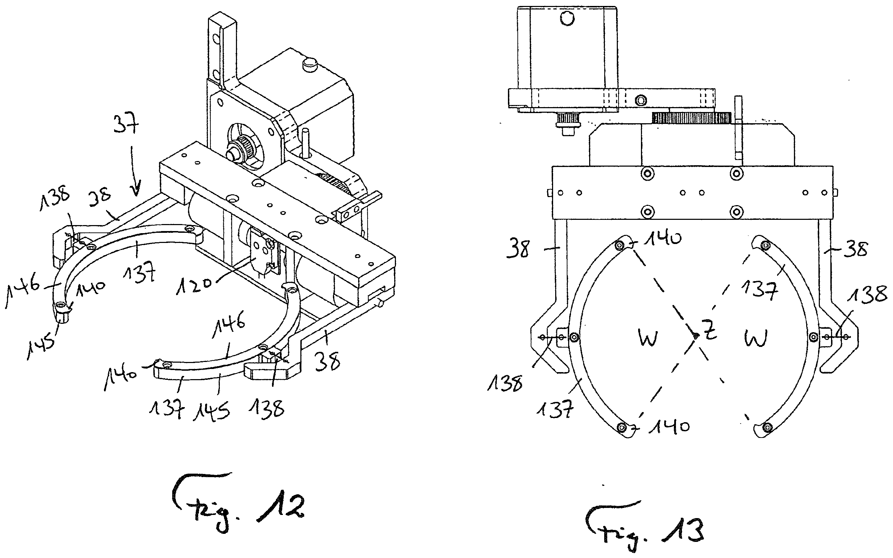

Bei einer bevorzugten Ausführung gemäss Figur 11 ist an der mindestens einen Übergabestation 4, wobei in dieser Figur zwei Übergabestationen 4 dargestellt sind, mindestens ein Fixierelement 100 für den Deckel 49 der Petrischale vorgesehen. Das Fixierelement ist so ausgeführt, dass durch dieses der Deckel gesteuert zeitweise fixierbar ist. Damit kann verhindert werden, dass ein allenfalls am Boden der Petrischale "klebender" Deckel vom Greifer mitgenommen wird, wenn der Greifer den Boden abhebt. Im gezeigten Beispiel sind an jeder Übergabestation 4 bzw. an deren Transfertisch 26 jeweils zwei Fixierelemente 100 und 101 angeordnet, welche ähnlich wie die Finger des Greifers für den Boden der Petrischale, den Deckel an seinem Rand angetrieben und gesteuert kontaktieren können und ihn damit fixieren. In der Figur sind die Antriebe 102 und 103 für die Fixierelemente 100 bzw. 101 ersichtlich, welche durch die Steuerung der Vorrichtung so betätigt werden, dass die Fixierelemente den Deckel festhalten bzw.fixieren, wenn der Greifer den Boden greift und abhebt. Die Fixierelemente sind bevorzugt bogenförmig und sie können in der Horizontalebene bzw. in einer Ebene koplanar zur Ebene des Transfertischs schwenkbar sein, um sich an den Deckel anpassen zu können. Dabei kann eine Schwenkbarkeit mit einer Blattfeder bewirkt werden, wie dies nachfolgend bei bevorzugten Fingern des Greifers bei den Figuren 12 und 13 noch erläutert wird.

In der Figur 11 ist ferner gezeigt, dass die Transfervorrichtung 3 für die Übergabestation 4, insbesondere für beide Übergabestationen 4, und den Greifer 36 eine gemeinsame Trägerplatte 110 aufweist, an welcher die Übergabestation 4 oder die Übergabestationen 4 und der Greifer 36 angeordnet sind, wobei dies insbesondere eine einstückige Trägerplatte und insbesondere L-förmige Trägerplatte ist. Dies ergibt eine sehr einfache Montage und hält die genannten Teile der Vorrichtung auf einfache Weise in genau definierter Lage zueinander, die sich nicht verstellen kann, was für die Wiederholgenauigkeit der Abläufe wichtig ist.

Bevorzugt ist es weiter, dass die Transfervorrichtung 3 im Bereich des Greifers 36 einen gesteuert zeitweise abschaltbaren optischen Sensor 120 aufweist, der zur Detektion des Vorhandenseins bzw. Nichtvorhandenseins der Petrischale im Greifer ausgestaltet und angeordnet ist. Der Sensor ist für die Abgabe von entsprechenden Signalen an die Steuerung der Vorrichtung vorgesehen. Die Abschaltung des optischen Sensors durch die Steuerung ermöglicht es, eine Störung der Inspektion der Petrischale durch den Lichteinfluss des Sensors zu vermeiden.

Bevorzugt ist weiter an jeder Übergabestation 4 ein Sensor 125 für die Präsenz bzw. Nichtpräsenz des Deckels in der Übergabestation vorgesehen, was insbesondere für die Steuerung der Fixierelemente 100 und 101 vorgesehen ist.

Die Vorrichtung ist bevorzugt so ausgebildet dass beim Greifer 36 dessen Grundstellung der greifende Zustand ist, so dass bei einer Störung, z.B. bei einem kurzzeitigen Stromunterbruch, das vom Greifer gehaltene Laborobjekt bzw. der Boden der Petrischale trotzdem festgehalten wird. Der Antrieb des Greifers wirkt somit derart, dass er den Greifer zum Loslassen antreibt und dass zum Festhalten der Antrieb nicht aktiv zu sein braucht. Bei den Fixierelementen 100 und 101 ist demgegenüber die Grundstellung das Offen sein, so dass ein Laborobjekt jedenfalls abgelegt bzw. auf dem Tisch 26 platziert werden kann und nur das Fixieren des aktiven Antriebs bedarf.

An Hand der Figuren 12 und 13 werden bevorzugte Ausführungen des Greifers erläutert, wobei der Greifer 36 mindestens zwei Finger 37 aufweist, welche je an einem Schaft 38 angeordnet sind, wobei die Schäfte gesteuert angetrieben aufeinander zu und voneinander weg bewegbar sind, wie dies schon erläutert worden ist. Ersichtlich ist, dass jeder Finger 37 in einer Horizontalebene schwenkbar am zugehörigen Schaft befestigt ist, was durch die gebogenen Pfeile in Figur 12 symbolisiert ist. Dabei ist es insbesondere vorgesehen, dass jeder Finger sich selbständig in eine unverschwenkte zentrierte Grundstellung bewegen kann, was auf eine einfache Weise eine gute Anpassung an jede neue zu greifende Schale ermöglicht. Dies ist bevorzugt so gelöst, dass der jeweilige Finger 37 mittels einer vertikal stehenden Blattfeder 138 an einer Stelle am zugehörigen Schaft befestigt ist. Die Blattfeder erlaubt die Schwenkbewegung in der Horizontalebene und nur in der Horizontalebene und bewirkt die Rückstellung.

Auch bei dieser Ausführung sind die Finger 37 des Greifers 36 für eine im Wesentlichen punktförmige Berührung der Petrischale ausgestaltet und angeordnet , wobei insbesondere eine punktförmige Berührung mittels der Endbereiche 140 der Finger bevorzugt ist, wie dies in den Figuren ersichtlich ist. Dabei ist bevorzugt, dass der Greifer 36 derart ausgestaltet ist, dass die Grösse des Winkels W vom Zentrum Z des Greifbereichs zu den äussersten, zur Berührung der Petrischale vorgesehenen und angeordneten Berührungsteilen der Finger 37 des Greifers 36 80 Winkelgrad bis 120 Winkelgrad beträgt und vorzugsweise 85 Grad bis 100 Grad beträgt und vorzugsweise ca. 90 Grad beträgt. Insbesondere der Wert von ca. 90 Grad ist für ein sicheres Greifen und Festhalten und Loslassen bevorzugt.
Weiter ist es vorteilhaft für die Ausführung und die Wartung, wenn die Finger 37 des Greifers 36 von einem Basisteil 145 und einem an diesem lösbar befestigten Wechselteil 146 gebildet sind. Das Wechselteil ist dabei für die punktförmige Berührung ausgestaltet. Das Basisteil kann aus einem Leichtmetall, insbesondere Aluminium, gebildet sein und das Wechselteil aus Stahl, insbesondere Federstahl.

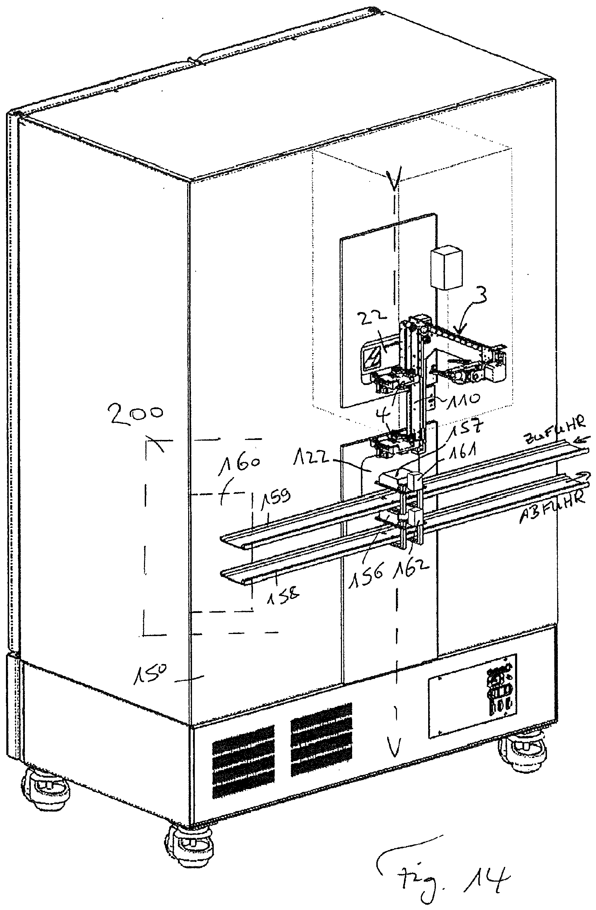

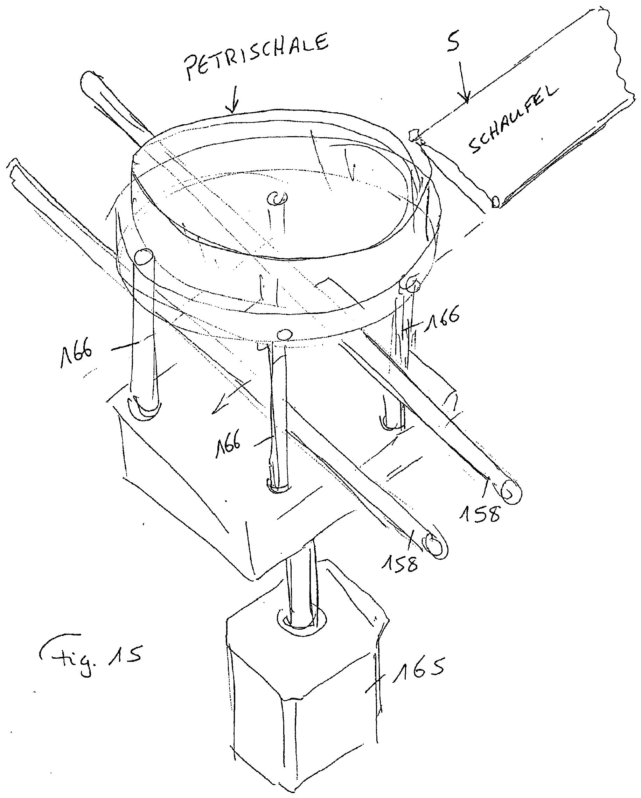

Bevorzugt ist die Vorrichtung in eine weitere Laborumgebung eingebunden und in diesem Zusammenhang ist es bevorzugt, wenn die Vorrichtung, wie in Figur 14 dargestellt, so ausgebildet ist, dass die Lagervorrichtung 1 an ihrer Wand 150, an welcher die Transfervorrichtung 3 befindlich ist, eine zweite Hilfstüre 122 aufweist, durch welche mittels der Umschlagsvorrichtung 5 und einer ausserhalb der Lagervorrichtung 1 an deren genannter Wand 150 befindlichen Transportanordnung 155 die Zufuhr bzw. Abfuhr von Petrischalen in bzw. aus der Lagervorrichtung bewirkbar ist, wobei die zweite Hilfstüre 122 insbesondere in derselben Vertikalachse V liegt wie die erste Hilfstüre 22, insbesondere unterhalb der ersten Hilfstüre. Dies erlaubt die Beschickung mit und die Entsorgung von Petrischalen im Zusammenspiel mit der Laborumgebung und der Inspektion und erbringt einen Geschwindigkeitsvorteil bei der Bewirtschaftung der Laborobjekte bzw. Petrischalen. Wie dargestellt, weist die Transportanordnung 155 zwei vertikal übereinander liegende steuerbar angetriebene Hebeeinrichtungen 156, 157 auf, durch welche Petrischalen von zwei übereinander angeordneten Förderbändern 158, 159 unabhängig voneinander abhebbar bzw. auf diesen absetzbar sind. Eine Hebeeinrichtung 156 ist beispielhaft in Figur 15 dargestellt. Gemäss Figur 14 dient diese Hebeeinrichtung zur Abfuhr von Petrischalen, welche von der Schaufel der Umschlagvorrichtung 5 durch die zweite Türe 122 aus der Lagervorrichtung ausgegeben werden. Durch deren unterseitigen Antrieb 165 können vier Säulen 166 angehoben werden und können die auf der Schaufel liegende Petrischale von unten her stützen, so dass die Schaufel der Umschlagvorrichtung 5 wieder zurückgezogen werden kann und die Petrischale auf den Säulen 166 aufliegt. Diese werden dann wieder abgesenkt werden und platzieren die Schale auf dem Förderband 158, das hier aus zwei separaten, synchron angetriebenen Riemen gebildet ist. Eine Steuerung und ein Antrieb 160 des Förderbands 158, welche Elemente zur übergeordneten Laborumgebung 200 gehören, die in Figur 14 angedeutet ist, führen dann die Petrischale z.B. zur Entsorgung ab. Für die andere Hebeeinrichtung 157 gilt für die Zufuhr von Petrischalen in die Lagervorrichtung 1 grundsätzlich dasselbe Vorgehen, wobei aber hier eine Petrischale von dem Förderband 159 zugeführt, von der Hebeeinrichtung 157 angehoben von der Schaufel der Umschlagvorrichtung 5 unterfahren und durch Absenken der Hebeeinrichtung von der Schaufel der Umschlagvorrichtung 5 übernommen und dann in die Lagervorrichtung 1 durch die zweite Türe eingefahren wird. Für die Steuerung dieser Vorgänge ist es bevorzugt, dass die Hebeeinrichtung jeweils einen Sensor aufweist, durch den das Vorhandensein bzw. Nichtvorhandensein einer Petrischale feststellbar ist.

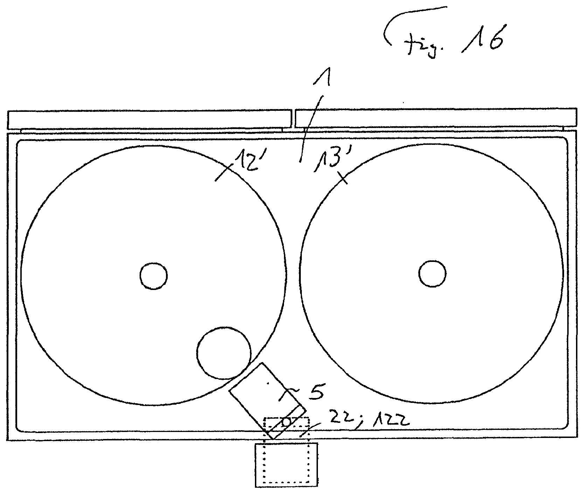

Gemäss Figur 16 ist es bevorzugt, wenn in der Lagervorrichtung zwei getrennt voneinander und nebeneinander angeordnete Karussells 12', 13' vorgesehen sind, welche von einer gemeinsamen Umschlagvorrichtung 5 bedienbar sind.

Die erläuterte Laborvorrichtung mit einer Vorrichtung erfindungsgemässen Vorrichtung wobei die Laborvorrichtung eine Steuerung und zwei übereinander liegende, steuerbar angetriebene Förderbänder 158, 159 aufweist, wobei die Förderbänder derart angeordnet und steuerbar sind, dass sie zum Zusammenwirken mit den Hebeeinrichtungen 156, 157 bestimmt sind, um Petrischalen in die bzw. aus der Lagervorrichtung (1) der Vorrichtung zuzuführen bzw. abzuführen ermöglicht eine besonders effiziente und rasche Handhabung der Petrischalen.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Lagervorrichtung, wobei die Lagervorrichtung Lagerschächte (14) aufweist, wobei jeder Lagerschacht (14) mehrere Lagerplätze zur Aufnahme jeweils eines Laborobjekts übereinander aufweist, wobei in einem Boden (67) jedes Lagerschachts (14) mindestens eine Öffnung (70) angeordnet ist, in welche mindestens ein Stift (71) einer Unterlage (12, 13) des Lagerschachts eingreift, **dadurch gekennzeichnet, dass** die Lagerschächte (14) von oben mittels federnder Zungen (73) gehalten sind.

2. Lagervorrichtung nach Anspruch 1, wobei die Lagervorrichtung mindestens ein Karussell (12, 13) aufweist, welches die Unterlage für die Lagerschächte bildet, wobei die Lagerschächte in mindestens einem Kreis auf dem mindestens einen Karussell (12, 13) angeordnet sind.

3. Lagervorrichtung nach Anspruch 2, wobei die Zungen (73) radial verlaufen und an einem Mittelbereich (74) der Lagervorrichtung befestigt sind.

4. Lagervorrichtung nach einem der Ansprüche 2 oder 3, wobei die Zungen (73) federnd vorgespannt sind und von oben auf die Lagerschächte (14) drücken.

5. Lagerschacht nach einem der vorangehenden Ansprüche, wobei die Lagerschächte (14) derart von oben mittels der federnden Zungen (73) gehalten sind, dass durch Deformation der Zunge ein Lagerschacht angehoben, von den Stiften gelöst und der Lagervorrichtung entnommen werden kann.

6. Lagerschacht, insbesondere zur Verwendung in der Lagervorrichtung nach einem der vorangehenden Ansprüche, wobei der Lagerschacht eine Rückwand (60) und zwei Seitenwände (61) aufweist, und wobei zumindest an den Seitenwänden (61) seitliche Ablagen (63) zur Aufnahme der Petrischalen (15) angeordnet sind, **dadurch gekennzeichnet, dass** die Rückwand (60) über jeweils einen Übergangsbereich (64) in jede Seitenwand (61) übergeht, wobei der Übergangsbereich (64) gerundet ist und/oder schräg zu der Rückwand (60) und der an ihn angrenzenden Seitenwand (61) verläuft.

7. Lagerschacht nach Anspruch 6, wobei der Zwischenbereich mit einem Krümmungsradius von mindestens 2 cm, vorzugsweise mindestens 5 cm, gerundet ist.

8. Lagerschacht nach Anspruch 7, wobei der Zwischenbereich einen im Wesentlichen ebenen Abschnitt aufweist, welcher senkrecht zu einer Längsachse des Lagerschachts eine Ausdehnung von mindestens 2 cm aufweist.

9. Lagerschacht nach einem der Ansprüche 6 bis 8, wobei auch an der Rückwand Ablagen (63) zur Aufnahme der Petrischalen (15) angeordnet sind.

10. Vorrichtung zur Lagerung und Handhabung von Petrischalen (15), wobei die Petrischalen (15) einen Boden (48) und einen Deckel (49) aufweisen, wobei an einem Umfang des Bodens (48) eine Bodenwand (53) angeordnet ist, umfassend
eine Lagervorrichtung (1) zum Lagern der Petrischalen (15) mit dem Deckel (49) nach unten,
eine Inspektionsvorrichtung (2) zum automatisierten Inspizieren von Petrischalen (15) ohne Deckel (49) und
eine Transfervorrichtung (3) zum Transferieren der Petrischalen (15) zwischen der Lagervorrichtung (1) und der Inspektionsvorrichtung (2),
wobei die Transfervorrichtung (3) einen Greifer (36) aufweist, mit welchem jeweils eine Petrischale (15) ohne den Deckel (49) lateral an der Bodenwand (53) ergreifbar sind, und
- wobei die Lagervorrichtung nach einem der Ansprüche 1 bis 5 ausgestaltet ist und/oder
- wobei sie einen Lagerschacht nach einem der Ansprüche 6 bis 9 aufweist.
